# EUROPÄISCHE PATENTSCHRIFT

(11) **EP 0 763 535 B1**
(45) Veröffentlichungstag und Bekanntmachung des Hinweises auf die Patenterteilung: **27.11.2002**
(21) Anmeldenummer: 96112990.5
(22) Anmeldetag: 13.08.1996
(51) Int. Cl.: C07D 413/06, A61K 31/42

(54) **Piperidinylmethyloxazolidin-2-on Derivate, deren Herstellung und deren Verwendung als ZNS wirksame Verbindungen**
Piperidinylmethyloxazolidin-2-one derivatives, their preparation and their use as pharmacological active CNS compounds
Dérivés de pipéridinylméthyloxazolidin-2-one, leur préparation et leur utilisation comme composés pharmacologiques actifs sur le système nerveux central

(30) Priorität: 25.08.1995 DE 19531321
(43) Veröffentlichungstag der Anmeldung: 19.03.1997
(73) Patentinhaber: MERCK PATENT GmbH, 64293 Darmstadt (DE)
(72) Erfinder: Prücher, Helmut, 64646 Heppenheim (DE); Bartoszyk, Gerd, 64331 Weiterstadt (DE)

(56) Entgegenhaltungen:
- EP-A- 0 300 272
- EP-A- 0 443 197
- EP-A- 0 635 505

## Beschreibung

Die Erfindung betrifft neue Piperidinylmethyloxazolidin-2-on-derivate, ausgewählt aus der Gruppe
a) (5S)-(-)-5-[4-(4-Fluorbenzyl)-1-piperidylmethyl]-3-(4-methoxyphenyl)-oxazolidin-2-on,
b) (5S)-(-)-5-[4-(4-Acetylaminobenzyl)-1-piperidylmethyl]-3-(4-methoxyphenyl)-oxazolidin-2-on,
c) (5S)-(-)-5-[4-(4-Aminobenzyl)-1-piperidylmethyl]-3-(4-methoxyphenyl)-oxazolidin-2-on,
d) (5S)-(-)-5-[4-(4-Acetylaminobenzyl)-1-piperidylmethyl]-3-phenyloxazolidin-2-on,
e)(5S)-(-)-5-[4-(4-Aminobenzyl)-1-piperidinylmethyl]-3-(4-fluorphenyl)-oxazolidin-2-on,
f) (5S)-(-)-5-[4-(4-Acetylaminobenzyl)-1-piperidylmethyl]-3-(4-fluorphenyl)-oxazolidin-2-on,
g) (5S)-(-)-5-[4-(4-Acetylaminobenzyl)-1-piperidylmethyl]-3-(4-chlorphenyl)-oxazolidin-2-on,
h) (5S)-(-)-5-[4-(4-Aminobenzyl)-1-piperidinylmethyl]-3-(4-chlorphenyl)-oxazolidin-2-on,
i) (5S)-(-)-5-[4-(4-Aminobenzyl)-1-piperidinylmethyl]-3-phenyloxazolidin-2-on,
sowie deren physiologisch unbedenklichen Salze. Die Erfindung betrifft auch deren Verwendung als psychopharmakologisch wirksame Substanzen.

Ähnliche Verbindungen mit einem Piperidinylmethyloxazolidin-2-on-Grundgerüst sind in EP 0 300 272 A2 beschrieben. Die Verbindung nach Anspruch 1a) ist als Auswahlerfindung zu EP 0 300 272 A2 zu betrachten.

In der Anmeldung DE 4005371 A1 sind neben einer großen Zahl möglicher anderer Verbindungen auch Piperidinylalkyloxazolidinone allgemein als pharmazeutisch wirksame Verbindungen beschrieben, in denen ein am Stickstoff substituierter Oxazolidinonring in 5-Stellung entweder über eine Ethyl- oder eine Propylgruppe an den Stickstoff eines ebenfalls substituierten Piperidinrings gebunden ist. Gemäß dieser Anmeldung wurden für solche Verbindungen Hinweise auf eine das Zentralnervensystem beeinflussende Wirkung gefunden.

Auch in der Anmeldung DE 43 24 393 A1 werden Piperidinylalkyloxazolidinone beschrieben, die eine das Zentralnervensystem beeinflussende , insbesondere neuroleptische Wirkung aufweisen, ohne daß eine nennenswerte kataleptische Wirkung auftritt. In diesem Fall handelt es sich um Piperidinderivate, die in 4-Stellung durch Aryloxy- bzw. Arylthiogruppen substituiert sind.

Der Erfindung lag daher die Aufgabe zugrunde, neue Verbindungen zur Verfügung zu stellen, die zur Herstellung von Arzneimitteln verwendet werden können, jedoch gegenüber den bereits bekannten Wirkstoffen ein ausgeprägteres Wirkspektrum aufweisen und selektiv auf das Zentralnervensystem wirken, nebenwirkungsarm sind, gleichzeitig aufgrund einer geänderten Struktur in möglichst geringer Dosis verabreicht werden können und dabei kein oder nur ein sehr geringes Abhängigkeitspotential aufweisen.

Es wurde nun gefunden, daß die Verbindungen nach Anspruch 1und ihre physiologisch unbedenklichen Salze bei guter Verträglichkeit wertvolle pharmakologische Eigenschaften besitzen. Sie beeinflussen insbesondere das Zentralnervensystem und weisen sedierende, tranquillierende, neuroleptische und/oder antidepressive Wirkungen auf, ohne daß eine nennenswerte kataleptische Wirkung nachweisbar ist.

Gegenstand der Erfindung sind daher die neuen Piperidinylmethyloxazolidin-2-on-derivate nach Anspruch 1 sowie ihre Salze und deren Verwendung als pharmakologisch wirksame Substanzen.

Im einzelnen haben die Verbindungen nach Anspruch 1 sowie ihre Salze eine dämpfende Wirkung auf das Verhalten bei Mäusen (Methodik vgl. Irwin, Psychopharmacologica 13 81968), 222-257). Sie hemmen bei Mäusen das durch Apomorphin induzierte Kletterverhalten (Methodik vgl. Costall et al., European J. Pharmacol. 50 (1968), 39 - 50) oder induzieren contralaterales Drehverhalten in Hemiparkinson-Ratten (feststellbar nach der Methode von Ungerstedt et al., Brain Res. 24 (1970), 485-493), ohne daß nennenswerte kataleptische Nebenwirkungen auftreten (Methodik vgl. Dolini-Stola, Pharmakopsychiat. 6 (1973), 189 - 197). Auch hemmen diese Wirkstoffe die Bindung von tritiierten Dopaminagonisten und - antagonisten an striäre Rezeptoren (feststellbar nach der Methode von Schwarcz et al., J. Neurochemistry 34 (1980), 772-778, und Creese et al., European J. Pharmacol. 46 (1977), 377-381). Weiterhin hemmen die Verbindungen den Zungen-Kieferreflex bei der narkotisierten Ratte (feststellbar in Anlehnung an die Methoden von Barnett et al., European J. Pharmacol. 21(1973), 178-182, und von Ilhan et al., European J. Pharmacol. 33 (1975), 61-64). Daneben sind analgetische und blutdrucksenkende Wirkungen nachweisbar; und zwar wird bei kathetertragenden wachen, spontan hypertonen Ratten (Stamm SHR/NiH-MO//CHB-EMD) der direkt gemessene arterielle Blutdruck nach intragastraler Gabe der Wirkstoffe gesenkt (Methodik vgl. Weeks und Jones, Proc. Soc. Exptl. Biol. Med. 104 (1960), 646-648).

Aufgrund dieser Ergebnisse der Untersuchungen hat sich gezeigt, daß die Verbindungen nach Anspruch 1 sowie ihre physiologisch unbedenklichen Säureadditionssalze als Arzneimittelwirkstoffe aber auch als Zwischenprodukte zur Herstellung weiterer Arzneimittelwirkstoffe verwendet werden können.

Hergestellt werden können die Verbindungen nach Anspruch 1, wie in Beispiel 1 beschrieben.

Die Herstellung der Verbindungen nach Anspruch 1 erfolgt im übrigen nach an sich bekannten Methoden, wie sie in der Literatur (z. B. in den Standardwerken wie Houben-Weyl, Methoden der Organischen Chemie, Georg-Thieme-Verlag; J. March, Advanced Organic Chemistry 3rd. Ed. (1984) oder Organic Reactions, beide John Wiley & Sons, Inc. New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen nach Anspruch 1 umsetzt.

Die Umsetzung zu Verbindungen nach Anspruch 1 verläuft nach Methoden, wie sie für die Alkylierung von Aminen aus der Literatur bekannt sind. Man kann beispielsweise die Ausgangsverbindungen direkt miteinander verschmelzen, und zwar in Abhängigkeit von ihren Eigenschaften gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z. B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetallhydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylamin, Pyridin oder Chinolin oder eines Überschusses der Aminkomponente bzw. der Verbindung der Formel III oder IIIa kann günstig sein. Die Reaktionstemperatur liegt je nach den angewendeten Bedingungen zwischen etwa 0 und 150 °C, normalerweise zwischen 20 und 130 °C.

Verbindungen nach Anspruch 1 können als Racemate oder, falls optisch aktive Ausgangsverbindungen eingesetzt werden, auch in optisch aktiver Form erhalten werden. Erhaltene Racemate können, falls erwünscht, nach an sich bekannten Methoden physikalisch oder chemisch aufgetrennt werden. Vorzugsweise werden aus den Racematen durch chemische Umsetzung mit einem optisch aktiven Trennmittel Diastereomere gebildet. Als Trennmittel eignen sich z.B. optisch aktive Säuren, wie die D- und L-Formen der Weinsäure, Dibenzoylweinsäure, Diacetylweinsäure, Camphersulfonsäure, Mandelsäure, Äpfelsäure oder Milchsäure. Die verschiedenen Formen der Diastereomere können in an sich bekannter Weise, z. B. durch fraktionierte Kristallisation, getrennt werden und die optisch aktiven Verbindungen nach Anspruch 1 in an sich bekannter Weise aus den Diastereomeren in Freiheit gesetzt werden.

Eine erhaltene Base nach Anspruch 1 kann mit einer Säure in das dazugehörige Säureadditionssalz überführt werden. Hierzu eignen sich insbesondere Säuren, die physiologisch unbedenkliche Salze liefern. Als anorganische Säuren können zu diesem Zweck Schwefelsäure, Halogenwasserstoffsäuren wie HCI, HBr, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure, Säureadditionssalze, die physiologisch nicht unbedenklich sind, können sich zur Isolierung und Aufreinigung von Basen der Formel I eignen.

Die Verbindungen nach Anspruch 1 und ihre physiologisch unbedenklichen Salze können daher zur Herstellung pharmazeutischer Präparate verwendet werden, indem man sie zusammen mit mindestens einem Träger- oder Hilfsstoff und, falls erwünscht, mit einem oder mehreren weiteren Wirkstoffen in die geeignete Dosierungsform bringt.

Die so erhaltenen Zubereitungen können als Arzneimittel in der Human- oder Veterinärmedizin eingesetzt werden.

Als Trägersubstanzen kommen organische oder anorganische Stoffe in Frage, die sich für die enterale (z. B. orale oder rektale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Cellulose, Magnesiumstearat, Talk oder Vaseline, Glycerintriacetat und andere Fettsäureglyceride, Sojalecithin.

Zur oralen Anwendung dienen insbesondere Tabletten, Dragees, Kapseln, Sirupe, Säfte oder Tropfen. Von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wäßrige Lösungen, ferner Suspensionen, Emulsionen oder Implantate, sowie für die topische Anwendung Salben, Cremes oder Puder.

Die erfindungsgemäß beanspruchten Wirkstoffe können auch lyophilisiert und die erhaltenen Lyophilisate z. B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z. B. ein oder mehrere Vitamine, Diuretika, Antiphlogistika.

Die Verbindungen nach Anspruch 1 und ihre physiologisch unbedenklichen Salze können zur therapeutischen Behandlung des menschlichen oder tierischen Körpers, insbesondere zur Bekämpfung von Krankheiten verwendet werden. Sie sind in der Behandlung der Schizophrenie, von psychoreaktiven Störungen und Psychopathien, Depressionen, schweren chronischen Schmerzen, und von Krankheiten, die mit erhöhtem Blutdruck einhergehen, wirksam. Auch können die Verbindungen bei der Behandlung extrapyramidaler Störungen Anwendung finden. Die erfindungsgemäßen Verbindungen sind als atypische Neuroleptika wirksam, zeigen dabei jedoch vorteilhafter Weise keine nennenswerten kataleptischen Nebenwirkungen auf.

Die erfindungsgemäßen Verbindungen nach Anspruch 1 und ihre physiologisch unbedenklichen Salze werden in der Regel in Analogie zu anderen bekannten, für die beanspruchten Indikationen im Handel erhältlichen Präparaten (Thioridazin, Haloperidol) verabreicht, vorzugsweise in Dosierungen zwischen etwa 0,1 mg und 500 mg, insbesondere zwischen 0,2 und 50 mg pro Dosierungseinheit. Die tägliche Dosierung liegt vorzugsweise zwischen etwa 0.002 und 20 mg/kg, insbesondere 0,2 und 0,4 mg/kg Körpergewicht.

Die spezielle Dosis für jeden einzelnen Patienten hängt jedoch von den verschiedensten Faktoren ab, beispielsweise von der Wirksamkeit der eingesetzten speziellen Verbindung, vom Alter, Körpergewicht, allgemeinem Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachstehenden Beispielen bedeutet "übliche" Aufarbeitung: Man gibt, falls erforderlich, Wasser hinzu, extrahiert mit Dichlormethan, trennt ab, trocknet die organische Phase über Natriumsulfat, filtriert, dampft ein und reinigt durch Chromatographie an Kieselgel und/oder durch Kristallisation. Alle Temperaturen sind in °C angegeben und die [α]_{D}-Werte bei 20 °C in Dimethylsulfoxid gemessen.

### Beispiele

### Beispiel 1

Eine Lösung, bestehend aus 4,92 g (5R)-(-)-5-(Methansulfonsäureoxymethyl)-3-p-fluorphenyl-oxazolidin-2-on, 65 ml Acetonitril, 4,70 g 4-(4-Aminobenzyl)-piperidin [herstellbar aus 4-(4-Nitrobenzyl)-pyridin durch Hydrierung der Nitrogruppe zu NH₂ und des Pyridinringes zum Piperidinring in Gegenwart eines Palladiumkatalysators in Eisessig] und 4,43 g Natriumhydrogencarbonat, wird für die Dauer von 26 Stunden unter Rückflußbedingungen gerührt. Anschließend wird das Reaktionsgemisch mit 100 ml Dichlormethan verdünnt, mehrmals mit geringen Mengen Wasser extrahiert, getrocknet. Nach dem Trocknen wird das Lösungsmittel abdestilliert und das erhaltene Produkt chromatographisch an einer Kieselgelsäule gereinigt. Auf diese Weise wird das Reaktionsprodukt als farbloses Harz erhalten, das auskristallisiert wird.
Ausbeute: 3,18 g (5S)-(-)-5-[4-(4-Aminobenzyl)-1-piperidinylmethyl]-3-(4-fluorphenyl)-oxazolidin-2-on (48,8 % der Theorie),
Fp. 95-99 °C
[α]_{D}²⁰ = -24,5° (DMSO)

Analog herstellbar sind
aus 4-(4-Acetylaminobenzyl)-piperidin [herstellbar aus 4-(4-Nitrobenzyl)-pyridin durch Hydrierung der Nitrogruppe zu NH₂ in Gegenwart eines Nickelkatalysators (Raney-Nickel) zu 4-(4-Aminobenzyl)-pyridin, Acetylierung mit Essigsäureanhydrid/Triethylamin zu 4-(Acetylaminobenzyl)-pyridin und anschließende Hydrierung des Pyridinrings in Gegenwart eines Palladiumkatalysators in Eisessig]
und (5R)-(-)-5-(Methansulfonsäureoxymethyl)-3-(p-fluorphenyl)-oxazolidin-2-on
(5S)-(-)-5-[4-(4-Acetylaminobenzyl)-1-piperidinylmethyl]-3-(4-fluorphenyl)-oxazolidin-2-on
Fp. 177 - 179 °C
[α]_{D}²⁰ = -23,6° (DMSO)
aus 4-(4-Fluorbenzyl)-piperidin
und (5R)-(-)-5-(Methansulfonsäureoxymethyl)-3-(4-methoxyphenyl)-oxazolidin-2-on
(5S)-(-)-5-[4-(4-Fluorbenzyl)-piperidinylmethyl]-3-(4-methoxyphenyl)-oxazolidin-2-on
FP. 203-205 °C
[α]_{D}²⁰ = -27,7° (DMSO)
aus 4-(4-Acetylaminobenzyl)-piperidin
und (5R)-(-)-5-(Methansulfonsäureoxymethyl)-3-(4-methoxyphenyl)-oxazolidin-2-on
(5S)-(-)-5-[4-(4-Acetylaminobenzyl)-1-piperidinylmethyl]-3-(4-methoxyphenyl)-oxazolidin-2-on
Fp. 204 - 206 °C
[α]_{D}²⁰ = -25,8° (DMSO)
aus 4-(4-Aminobenzyl)-piperidin
und (5R)-(-)-5-(Methansulfonsäureoxymethyl)-3-(4-methoxyphenyl)-oxazolidin-2-on
(5S)-(-)-5-[4-(4-Aminobenzyl)-1-piperidinylmethyl]-3-(4-methoxyphenyl)-oxazolidin-2-on
Fp. 126 - 128 °C
[α]_{D}²⁰ = -27,8° (DMSO)
aus 4-(4-Acetylaminobenzyl)-piperidin
und (5R)-(-)-5-(Methansulfonsäureoxymethyl)-3-phenyl-oxazolidin-2-on
(5S)-(-)-5-[4-(4-Acetylaminobenzyl)-1-piperidinylmethyl]-3-phenyloxazolidin-2-on
Fp. 199 - 201 °C
[α]_{D}²⁰ = -24,3° (DMSO)
aus 4-(4-Acetylaminobenzyl)-piperidin
und (5R)-(-)-5-(Methansulfonsäureoxymethyl)-3-(4-chlorphenyl)-oxazolidin-2-on
(5S)-(-)-5-[4-(4-Acetylaminobenzyl)-1-piperidinylmethyl]-3-(4-chlorphenyl)-oxazolidin-2-on
Fp. 221-223 °C
[α]_{D}²⁰ = -27,4° (DMSO)
aus 4-(4-Aminobenzyl)-piperidin
und (5R)-(-)-5-(Methansulfonsäureoxymethyl)-3-(4-chlorphenyl)-oxazolidin-2-on
(5S)-(-)-5-[4-(4-Aminobenzyl)-1-piperidinylmethyl]-3-(4-chlorphenyl)-oxazolidin-2-on
Fp. 146 - 149 °C
[α]_{D}²⁰ = -29,8° (DMSO)
aus 4-(4-Aminobenzyl)-piperidin
und (5R)-(-)-5-(Methansulfonsäureoxymethyl)-3-phenyloxazolidin-2-on
(5S)-(-)-5-[4-(4-Aminobenzyl)-1-piperidinylmethyl]-3-phenyloxazolidin-2-on
Fp. 148 - 150 °C
[α]_{D}²⁰ = -28,5° (DMSO)

Die nachfolgenden Beispiele betreffen pharmazeutische Zubereitungen:

### Beispiel A: Injektionsgläser

Eine Lösung von 100 g eines Wirkstoffs nach Anspruch 1 und 5 g Dinatriumhydrogenphosphat werden in 3 l zweifach destilliertem Wasser mit 2 n Salzsäure auf pH 6.5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffs nach Anspruch 1 mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffs nach Anspruch 1, 9.38 g NaH₂PO₄^{·}2H₂O, 28.48 g Na₂HPO₄^{·}12H₂O und 0.1 g Benzalkoniumchlorid in 940 ml zweifach destilliertem Wasser. Man stellt auf pH 6.8 ein, füllt auf 1 l auf und sterilisiert durch Bestrahlung.

### Beispiel D: Salbe

Man mischt 500 mg eines Wirkstoffs nach Anspruch 1 mit 99.5 g Vaseline unter aseptischen Bedingungen.

### Beispiel E: Tabletten

Ein Gemisch von 1 kg Wirkstoff nach Anspruch 1, 4 kg Laktose, 1.2 kg Kartoffelstärke, 0.2 kg Talk und 0.1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragent und Farbstoff überzogen werden.

### Beispiel G: Kapseln

2 kg Wirkstoff nach Anspruch 1 werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff nach Anspruch 1 in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen ausgewählt aus der Gruppe
a) (5S)-(-)-5-[4-(4-Fluorbenzyl)-1-piperidylmethyl]-3-(4-methoxyphenyl)-oxazolidin-2-on,
b) (5S)-(-)-5-[4-(4-Acetylaminobenzyl)-1-piperidylmethyl]-3-(4-methoxyphenyl)-oxazolidin-2-on,
c) (5S)-(-)-5-[4-(4-Aminobenzyl)-1-piperidylmethyl]-3-(4-methoxyphenyl)-oxazolidin-2-on,
d) (5S)-(-)-5-[4-(4-Acetylaminobenzyl)-1-piperidylmethyl]-3-phenyloxazolidin-2-on,
e) (5S)-(-)-5-[4-(4-Aminobenzyl)-piperidinylmethyl]-3-(4-fluorphenyl)-oxazolidin-2-on,
f) (5S)-(-)-5-[4-(4-Acetylaminobenzyl)-1-piperidylmethyl]-3-(4-fluorphenyl)-oxazolidin-2-on,
g) (5S)-(-)-5-[4-(4-Acetylaminobenzyl)-1-piperidylmethyl]-3-(4-chlorphenyl)-oxazolidin-2-on,
h) (5S)-(-)-5-[4-(4-Aminobenzyl)-1-piperidinylmethyl]-3-(4-chlorphenyl)-oxazolidin-2-on
i) (5S)-(-)-5-[4-(4-Aminobenzyl)-1-piperidinylmethyl]-3-phenyloxazolidin-2-on,
sowie deren Salze.

2. Verfahren zur Herstellung pharmazeutischer Zubereitungen, **dadurch gekennzeichnet, daß** man eine Verbindung gemäß Anspruch 1 und/oder eines ihrer physiologisch unbedenklichen Salze zusammen mit mindestens einem festen, flüssigen oder halbflüssigen Träger- und/oder Hilfsstoff in eine geeignete Dosierungsform bringt.

3. Pharmazeutische Zubereitung, **gekennzeichnet durch** einen Gehalt an mindestens einer Verbindung gemäß Anspruch 1 und/oder einem ihrer physiologisch unbedenklichen Salze.

4. Verwendung von Verbindungen nach Anspruch 1 oder von deren physiologisch unbedenklichen Salzen zur Herstellung eines Arzneimittels.

5. Verwendung von Verbindungen nach Anspruch 1 oder von deren physiologisch unbedenklichen Salzen zur Herstellung von Arzneimitteln mit psychopharmakologischer Wirkung.

6. Verwendung von Verbindungen nach Anspruch 1 oder von deren physiologisch unbedenklichen Salzen zur Herstellung von Arzneimitteln mit atypischer neuroleptischer Wirkung.

## Claims

1. Compounds selected from the group consisting of
a) (5S)-(-)-5-[4-(4-fluorobenzyl)-1-piperidylmethyl]-3-(4-methoxyphenyl)oxazolidin-2-one,
b) (5S)-(-)-5-[4-(4-acetylaminobenzyl)-1-piperidylmethyl]-3-(4-methoxyphenyl)oxazolidin-2-one,
c) (5S)-(-)-5-[4-(4-aminobenzyl)-1-piperidylmethyl]-3-(4-methoxyphenyl)oxazolidin-2-one,
d) (5S)-(-)-5-[4-(4-acetylaminobenzyl)-1-piperidylmethyl]-3-phenyloxazolidin-2-one,
e) (5S)-(-)-5-[4-(4-aminobenzyl)piperidinylmethyl]-3-(4-fluorophenyl)oxazolidin-2-one,
f) (5S)-(-)-5-[4-(4-acetylaminobenzyl)-1-piperidylmethyl]-3-(4-fluorophenyl)oxazolidin-2-one,
g) (5S)-(-)-5-[4-(4-acetylaminobenzyl)-1-piperidylmethyl]-3-(4-chlorophenyl)oxazolidin-2-one,
h) (5S)-(-)-5-[4-(4-aminobenzyl)-1-piperidinylmethyl]-3-(4-chlorophenyl)oxazolidin-2-one,
i) (5S)-(-)-5-[4-(4-aminobenzyl)-1-piperidinylmethyl]-3-phenyloxazolidin-2-one,
and their salts.

2. Process for the production of pharmaceutical preparations, **characterized in that** a compound according to Claim 1 and/or one of its physiologically acceptable salts is brought into a suitable dose form together with at least one solid, liquid or semiliquid excipient and/or auxiliary.

3. Pharmaceutical preparation, **characterized in that** it contains at least one compound according to Claim 1 and/or one of its physiologically acceptable salts.

4. Use of compounds according to Claim 1 or of their physiologically acceptable salts for the production of a medicament.

5. Use of compounds according to Claim 1 or of their physiologically acceptable salts for the production of medicaments having psychopharmacological action.

6. Use of compounds according to Claim 1 or of their physiologically acceptable salts for the production of medicaments having atypical neuroleptic action.

## Revendications

1. Les composés choisis dans le groupe constitué par
a) la (5S)-(-)-5-[4-(4-fluorobenzyl)-1-pipéridylméthyl]-3-(4-méthoxyphényl)oxazolidin-2-one,
b) la (5S)-(-)-5-[4-(4-acétylaminobenzyl)-1-pipéridylméthyl]-3-(4-méthoxyphényl)oxazolidin-2-one,
c) la (5S)-(-)-5-[4-(4-aminobenzyl)-1-pipéridylméthyl]-3-(4-méthoxyphényl)oxazolidin-2-one,
d) la (5S)-(-)-5-[4-(4-acétylaminobenzyl)-1-pipéridylméthyl]-3-phényloxazolidin-2-one,
e) la (5S)-(-)-5-[4-(4-aminobenzyl)-pipéridinylméthyl]-3-(4-fluorophényl)oxazolidin-2-one,
f) la (5S)-(-)-5-[4-(4-acétylaminobenzyl)-1-pipéridylméthyl)-3-(4-fluorophényl)oxazolidin-2-one,
g) la (5S)-(-)-5-[4-(4-acétylaminobenzyl)-1-pipéridylméthyl]-3-(4-chlorophényl)oxazolidin-2-one,
h) la (5S)-(-)-5-[4-(4-aminobenzyl)-1-pipéridylméthyl]-3-(4-chlorophényl)oxazolidin-2-one,
i) la (5S)-(-)-5-[4-(4-aminobenzyl)-1-pipérldylméthyl]-3-phényloxazolidin-2-one,
ainsi que leurs sels.

2. Procédé pour la fabrication de préparations pharmaceutiques, **caractérisé en ce que** l'on met sous une forme de dosage appropriée un composé selon la revendication 1 et/ou l'un de ses sels physiologiquement acceptables, associés à au moins un support et/ou un adjuvant solide, liquide ou semi-liquide.

3. Préparation pharmaceutique **caractérisée en ce qu'**elle contient au moins un composé selon la revendication 1 et/ou l'un de ses sels physiologiquement acceptables.

4. Utilisation des composés selon la revendlcation 1 ou de leurs sels physiologiquement acceptables pour la fabrication d'un médicament.

5. Utilisation des composés selon la revendication 1 ou de leurs sels physiologiquement acceptables pour la fabrication de médicaments ayant une action psychopharmacologique.

6. Utilisation des composés selon la revendication 1 ou de leurs sels physiologiquement acceptables pour la fabrication de médicaments ayant une action neuroleptique atypique.
